# EUROPEAN PATENT SPECIFICATION

(11) **EP 2 380 570 B1**
(45) Date of publication and mention of the grant of the patent: **17.08.2016**
(21) Application number: 09833900.5
(22) Date of filing: 01.04.2009
(51) Int. Cl.: A61K 31/352, A61K 31/7048, A61P 3/10, A61P 13/12

(54) **PHARMACEUTICAL COMPOSITION FOR PREVENTING AND TREATING DIABETIC NEPHROPATHY AND PREPARATION METHOD THEREOF**
PHARMAZEUTISCHE ZUSAMMENSETZUNG ZUR VERHINDERUNG UND BEHANDLUNG DIABETISCHER NEPHROPATHIE SOWIE VERFAHREN ZU IHRER HERSTELLUNG
COMPOSITION PHARMACEUTIQUE DE PRÉVENTION ET TRAITEMENT D'UNE NÉPHROPATHIE DIABÉTIQUE ET SON PROCÉDÉ DE PRÉPARATION

(30) Priority: 16.01.2009 CN 200910036717
(43) Date of publication of application: 26.10.2011
(73) Proprietor: Guangzhou Consun Medicine R&D Co., Ltd., Guangzhou, Guangdong 510530 (CN)
(72) Inventor: ZHU, Quan, Guangzhou Guangdong 510530 (CN); SHI, Xinghua, Guangzhou Guangdong 510530 (CN); TANG, Dan, Guangzhou Guangdong 510530 (CN); ZHENG, Zhaoguang, Guangzhou Guangdong 510530 (CN); HE, Bao, Guangzhou Guangdong 510530 (CN); DUAN, Tingting, Guangzhou Guangdong 510530 (CN); GU, Fei, Guangzhou Guangdong 510530 (CN); CHENG, Huiquan, Guangzhou Guangdong 510530 (CN); HUANG, Xiaoling, Guangzhou Guangdong 510530 (CN); HUANG, Yanxia, Guangzhou Guangdong 510530 (CN); WANG, Rushang, Guangzhou Guangdong 510530 (CN)
(74) Representative: Corradini, Corrado
(86) International application number: PCT/CN2009/000352
(87) International publication number: WO 2010/081264

(56) References cited:
- CN-A- 1 403 452
- CN-A- 1 813 711
- CN-A- 1 985 876
- CN-A- 101 139 378
- KR-A- 20060 057 291
- US-A1- 2005 118 290
- US-A1- 2008 118 584
- SHEN P. ET AL.: 'Differential Effects of Isoflavones, from Astragalus Membranaceus and Pueraria Thomsonii, on the Activation of PPARalpha, PPARgamma, and A Adipocyte Differentiation in Vitro.' JOURNAL OF NUTRITION vol. 136, no. 4, 2006, pages 899 - 905, XP008139243
- DOU HUI ET AL.: 'Chemical Constituents of Huangqi Injection' NATURAL PRODUCT RESEARCH AND DEVELOPMENT vol. 14, no. 6, 2002, pages 14 - 17, XP008139245
- WANG GUOLI ET AL.: 'Observation of Recent Curative Effect of Astragalus Membranaceus Injection on Diagetic Nephropathy' CHINESE JOURNAL OF PRIMARY MEDICINE AND PHARMACY vol. 10, no. 1, January 2003, pages 38 - 39, XP008141638

## Description

### FIELD OF THE INVENTION

This invention relates to the field of traditional Chinese medicines, specifically the combination of calycosin (CAL) and calycosin-7-O-β-D-glucoside (CAG), for use in preventing and treating diabetic nephropathy (DN).

### BACKGROUND OF THE INVENTION

DN, a progressive kidney disease caused by angiopathy of capillaries in the kidney glomeruli, is one of the most severe complications of diabetes. DN is characterized by early mesangial cell proliferation, followed by accumulation of extracellular matrix proteins, which result in high filtration of renal glomeruli and albuminuria. Its major clinical symptoms are albuminuria, progressive kidney lesion, hypertension and oedema, which may result in end-stage renal failure, one of the leading causes of death. According to statistic data, about 30-40% diabetic people have renal lesion. It has become the primary and secondary cause of end-stage renal disease in the United State and Europe, respectively. In present, 15% Chinese people with end-stage renal disease are diabetics. However, with the incidence of diabetes continues to increase and the aging of the population, this figure will continue to rise.

Still now, there are few drugs with effective treatment effect towards DN, while hypoglycemic (angiotensin converting enzyme inhibitors, ACEI) and antihypertensive agents are widely used in the clinic practices, such as insulin and captopril. However, long-term application of these drugs will result in many serious side effects, *i.e.* hyperkaliemia, or even acute renal failure due to functional or organic renal artery stenosis. Therefore, development of novel drugs with safe and effective prevention and treatment of DN is very important.

DN is also known as diabetic glomerulosclerosis. In the renal glomerulus, the mesangial cell occupies a central position, and it constitutes approximately 30-40% of the total renal glomerular cell population. Glomerular mesangial cell is capable of a number of various physiological functions, *i.e.* structural support and secretion of mesangial extracellular matrix (ECM), response to and secretion of cytokines, phagocytosis of macromolecules and immune complexes. Mesangial cell proliferation, expansion and deposition of ECM are key pathological features in DN.

Radix Astragali is widely used as a traditional Chinese medicine. Its main indication is to disperse blood stasis and alleviate pain, to promote blood circulation and open channels, to clear away heat and relieve vexation, and so on. Recently, more and more experiments and clinic practices proved that the extract of Radix Astragali might have beneficial effects on the progress of DN. This finding is also consistent with the fact that Radix Astragali has widely been used for the treatment of diabetes mellitus in traditional Chinese medicine. At present, most researches remain focusing on the herb crude extracts, however, which bioactive components responding to the effects is still obscure. So, it is important to carry out further researches on it to develop modern traditional Chinese medicine preparations, with the characterizations of the clear structure and convenience of quality control, for preventing and treating DN.

Previous chemical investigations into Radix Astragali showed that isoflavonoid was one of the main chemical components, and it possessed various biological effects including free radical scavenger, regulation of the immune, anti-virus and anti-tumor activities. Calycosin (CAL) and calycosin-7-O-β-D-glucoside (CAG) are two major isoflavonoids in Radix Astragali. Patent CN01126608.2 reported that CAL and CAG showed anti-myocardial ischemia activity. Patent CN200510110641.3 reported that CAG could inhibit Coxsackie virus and treat viral myocarditis. In addition, they also possessed some other biological effects including antioxidant (Biomed Environ Sci, 2005, 18(5): 297-301), protective effects on vascular endothelial cell (Chin J Pharm, 2008, 43(8): 594-597; Pharmacol Clin Chin Mater Med, 2000, 16(4): 16-18), and calcium channel blocking activities (Acta Pharmacol Sin, 2006, 27(8): 1007-1012), modifying of red blood cells deformability (Nat Prod Res Develop, 1999, 6(2): 1-5), and alleviation of osteoarthritis (Osteoarthritis Cartilage, 2007, 15(9): 1086-1092).

As described above, they exhibited various biological effects, however, no information is available on the effects of CAL and CAG for preventing and treating DN.

US-A-2008 118584 discloses a composition for use in the treatment of diabetic nephropathy comprisingextracts of plants including Astragalus membranaceus.

### SUMMARY OF THE INVENTION

It is an object of the present invention to provide a pharmaceutical composition for the prevention and treatment of DN and a method for its preparation. The active ingredient of pharmaceutical composition is CAL and CAG.

To achieve the above object, the technical solution is as follows:
The pharmaceutical composition for preventing and treating DN comprises CAL and CAG as 0.1~99.5% by weight based on the total weight of the composition, and the conventional drug carrier, wherein the weight ratio of CAL to CAG is in a range from 1:5 to 5:1.

Preferably, the amount of inventive compound should be present between 5 to 90% by weight based on the total weight of the composition.

The drug carrier is conventional in the pharmaceutical field, for example, diluent or excipients such as water; fillers such as starch, sucrose; adhesives such as cellulose derivatives, algal acid, gelatin and polyvinylpyrrolidone; wetting agents such as glycerol; disintegrants such as agar, calcium carbonate and sodium bicarbonate; absorption enhancers such as quaternary ammonium compounds; surfactants such as palmityl alcohol; adsorption carrier such as kaolin and soap clay; lubricants such as talc, calcium and magnesium stearate, and polyethylene glycol. In addition, other auxiliary agents such as flavor agents and sweeteners can also be added.

The pharmaceutical composition can be prepared into conventional dosage forms by conventional methods, and the conventional dosage forms are primary oral preparations, for example, the solid dosage forms such as tablets, granules, pills, powders, or capsules, and liquid dosage forms such as water or oil suspension or elixir.

The desirable dose of the inventive compound or composition varies depending on the condition and the weight of the subject, severity, drug form, route and period of administration, and may be chosen by those skilled in the art. However, because the effective amount of the inventive compound for rat is 2-10mg/kg by weight/time, it is generally recommended to administer at the amount ranging 20-100mg/60kg by weight/times of the inventive compounds of the present invention according to the ratio of body surface area.

It is a further object of the present invention to provide the preparative method of the pharmaceutical composition comprising the mixture of CAL and CAG, and the conventional drug carrier.

CAL and CAG can be prepared by the procedure comprising the following steps:
A. Radix Astragali in pulverization is extracted with 80% ethanol under reflux and filter, and the alcohol extract is combined and then concentrated *in vacuo.* The concentrates was then refrigerated overnight at 4°C.
B. The supernatant obtained by step A is run on a macroporous resin column and eluted with water, and then 50% ethanol until no CAG is detected using TLC.
C. The 50% ethanol fraction solvent is concentrated, and then extracted using equivalent ethyl acetate, and the ethyl acetate extract is combined and then concentrated *in vacuo.*
D. The extract is chromatographied over silica gel, and eluted gradiently with a solvent system of chloroform-methanol, and the same fractions are combined by TLC analysis in order to crystallize, then the crystal is obtained with sucking filtration. Finally, CAL and CAG are obtained by recrystallization in methanol.

CAL and CAG can also be obtained from other traditional Chinese medicines according to the above method.

The present invention also provides the use of CAL and/or CAG in the manufacture of a medicament for the prevention and treatment of DN, including L II, III, IV type DN.

### ADVANTAGES OF THE INVENTION

The pharmaceutical composition in the present invention had the following useful significances:
1. The present invention discovered that CAL and/or CAG possessed a new medical use with prevention and treatment of DN through the pharmacological experiments, as well as provided a new drug candidate for patients with DN.
2. The effects of the pharmaceutical composition, with the characterizations of the clear structure and convenience of quality control, were better than the aqueous extract of Radix Astragali.
3. The pharmaceutical composition showed significant effects of treatment and prevention of glomerular damage induced by diabetes, and the diabetes itself because that it could significantly prevent the increase of serum creatinine, induce the urinary production and urine protein.
4. The pharmaceutical composition could be processed into various convenient oral dosage forms such as pills, granules, tablets and so on.

### BRIEF DESCRIPTION OF THE DRAWINGS

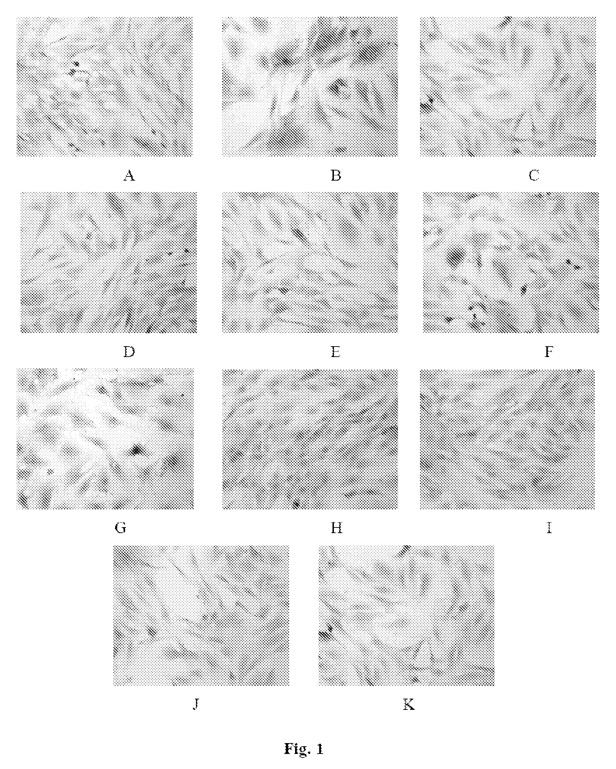

**Fig. 1** shows the effects of CAL and/or CAG on the ECM accrementition induced by high glucose (PAS, 200×)
   wherein A: normal glucose group; B: high glucose group (25mM); C: Radix Astragali group (RA, 50mg/ml aqueous extract); D: CAL group (100nM); E: CAL group (10nM); F: CAL group (1nM); G: CAG group (100nM); H: CAG group (10nM); I: CAG group (1nM); J: CAL:CAG group (10µg/ml:5µg/ml); K: CAL:CAG group (5µg/ml:10µg/ml).

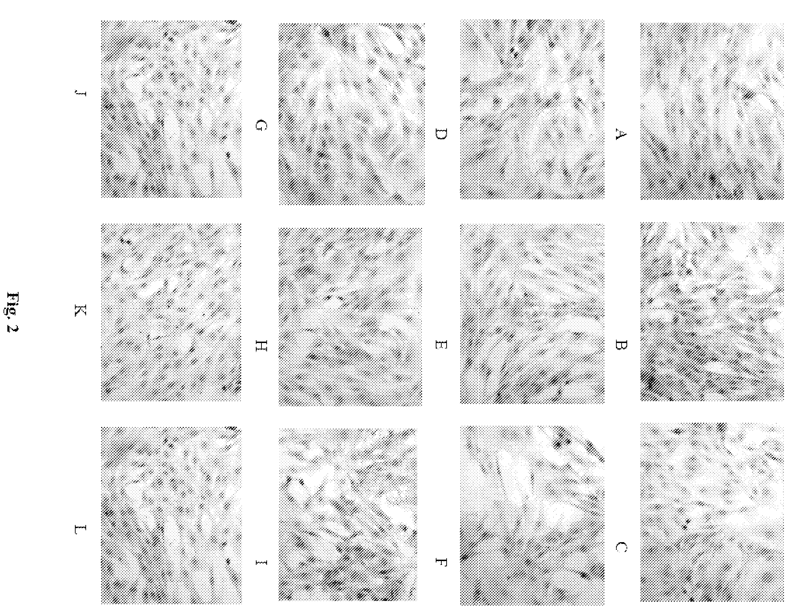

**Fig. 2** shows the effects of CAL and/or CAG on the TGFβ-1 expression induced by high glucose (200×)
   wherein A: normal glucose group; B: high glucose group (25mM); C: negative control group; D: CAL group (100nM); E: CAL group (10nM); F: CAL group (1nM); G: CAG group (100nM); H: CAG group (10nM); I: CAG group (1nM); J: Radix Astragali group (RA, 50mg/ml aqueous extract); K: CAL:CAG group (10µg/ml:5µg/ml); L: CAL:CAG group (5µg/ml:10µg/ml).

### DETAILED DESCRIPTION OF THE PREFERRED EMBODIMENT

### Examples

The present invention is more specifically explained by the following examples.

### Example 1 Preparation of CAL and CAG from Radix Astragali

The dried roots of *Astragalus membranaceus* var. *monghulicus* (10 kg) were extracted with 80% ethanol under reflux (80L×3) three times (2h, 1h, 1h). The alcohol extracts were combined and then concentrated *in vacuo.* The concentrates were dissolved in water and then refrigerated overnight at 4°C. The supernatant was run on a D101 macroporous resin column and eluted with water, and then 50% ethanol until no CAG was detected using TLC. The 50% ethanol fraction solvent was concentrated, then extracted 7 times using ethyl acetate, then the extracts were concentrated and chromatographied over silica gel (200~300 mesh), and was eluted gradiently with a solvent system of chloroform-methanol (50:1, 25:1, 12:1, 6:1, 3:1, 1.5:1, 1:1). The same fractions were combined to crystallize. Finally, white needle crystals (CAL) and powders (CAG) were obtained by recrystallization using methanol. Their structures were confirmed by NMR as well as MS, and the purity was more than 98% by HPLC analysis. Their structures were shown as follow:

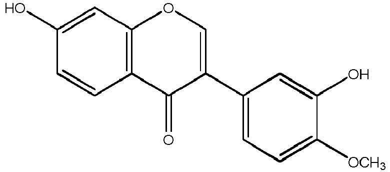

CAL: molecular weight: 284, molecular formula: C₁₆H₁₂O₅, chemical name: 3'-hydroxy-4'-methoxyisoflavone.

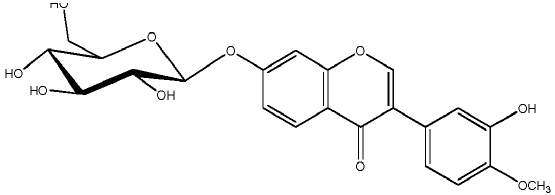

CAG: molecular weight:
446, molecular formula: C₂₂H₂₂O₁₀, chemical name: 3'-hydroxy-4'-methoxyisoflavone-7-O-β-D-glucopyranoside.

Hereinafter, the formulating methods and kinds of excipients will be described. The representative preparation examples were described as follows.

### Example 2 Preparation of granule

| | |
|---|---|
| CAG | 1mg |
| Starch | 500mg |
| Microcrystalline cellulose | 500mg |

Powder preparation was prepared by mixing above components and filling sealed package.

### Example 3 Preparation of tablet

| | |
|---|---|
| CAG | 20mg |
| Lactose | 127mg |
| Corn starch | 50mg |
| Magnesium stearate | 3mg |

Tablet preparation was prepared by mixing above components and entabletting.

### Example 4 Preparation of capsule

| | |
|---|---|
| CAL | 30mg |
| CAG | 70mg |
| Lactose | 40mg |
| Dextrin | 15mg |
| Starch | 45mg |
| 3%HPMC | appropriate |

Capsule preparation was prepared by mixing above components and filling gelatin capsule by conventional gelatin preparation method.

### Example 5 Preparation of granule

| | |
|---|---|
| CAG | 50mg |
| Starch | 400mg |
| Microcrystalline cellulose | 550mg |

Powder preparation was prepared by mixing above components and filling sealed package.

### Example 6 Preparation of capsule

| | |
|---|---|
| CAL | 120mg |
| CAG | 60mg |
| Dextrin | 10mg |
| Starch | 10mg |

Capsule preparation was prepared by mixing above components and filling gelatin capsule by conventional gelatin preparation method.

### Example 7 Preparation of tablet

| | |
|---|---|
| CAL | 200mg |
| HPMC | 2mg |

Tablet preparation was prepared by mixing above components and entabletting.

### Example 8 Preparation of tablet

| | |
|---|---|
| CAG | 200mg |
| HPMC | 2mg |

Tablet preparation was prepared by mixing above components and entabletting.

### Example 9 Preparation of capsule

| | |
|---|---|
| CAL | 80mg |
| CAG | 20mg |
| Lactose | 40mg |
| Dextrin | 15mg |
| Starch | 45mg |
| 3%HPMC | appropriate |

Capsule preparation was prepared by mixing above components and filling gelatin capsule by conventional gelatin preparation method.

In the present invention, CAL and/or CAG was used in the experiment to confirm the effects of preventing and treating DN, which was compared with the aqueous extract of Radix Astragali.

### Experiment 1 The effects of CAL and/or CAG on high glucose or AGEs-induced rat mesangial cell proliferation

Mesangial cell is the most active cell in the renal glomerulus, while the pathological change of mesangial cell occupies the central position in the development of DN, and it is also the target cell of DN. Thus, the effects of CAL and/or CAG on high glucose or AGEs-induced rat mesangial cell proliferation were investigated *in vitro.*

### 1. Materials and reagents

Rat mesangial cell line HBZY21 (Wuhan Institute of Cell Biology), new-born calf serum (Hangzhou Sijiqin Serum Factory), MTT (Amresco), dimethylsulfoxide (Shanghai Jiuqi Chemical Reagent Co. Ltd.), CO₂ incubator (NAPC05410, PERCISION SCIENTIFIC), XSZ-D2 inverted microscope (Chongqing Optical Instrument Factory), superclean bench (Suzhou Baishen network system Co. Ltd.), Microplate Spectrophotometer (SPECTRA max190, AD Co. Ltd. USA), clinical centrifuge (LDZ5-2, Beijing Clinical Centrifuge Factory).

### 2. Method

### 2.1 Experimental model

The mesangial cells in their exponential growth phase were cultured in 96-well plates at densities of 50000 cm⁻² in DMEM (5mM glucose) at 37°C in 5%CO2-95% air for 24h. After pre-incubation in DMEM supplemented without fetal calf serum for 24 h, cells were then treated with various concentrations of glucose or AGEs for 24h, 48h, 72h, 96h, respectively. The mesangial cell proliferation was measured by MTT and the results showed that the mesangial cell treated by 0.25mg/ml AGEs or 25 mM glucose for 24h was the best experimental model condition.

### 2.2 The effects on rat mesangial cells proliferation induced by high glucose

According to the aboved method, after pre-incubation in DMEM supplemented without fetal calf serum for 24 h, cells were pretreated with 25mM glucose, then were treated with various concentrations of reagents as indicated: normal glucose group (NG, 5.5 mM glucose); high glucose group (HG, 25 mM glucose); CAL group (CAL, 10, 100nM); CAG group (CAG, 10, 100nM); CAL+CAG group (CAL:CAG, 10µg/ml:5µg/ml, 5µg/ml:10µg/ml); Radix Astragali group (RA, 50 mg/ml aqueous extract). The plates were then incubated for 24 h. Proliferation was examined by MTT reduction assay. Experimental conditions were tested in sextuplicate (six wells of the 96-well plate per experimental condition).

### 2.3 The effects on rat mesangial cells proliferation induced by AGEs

Cells were pretreated with 0.25mg/mlAGEs, then were treated with various concentrations of reagents as indicated: normal glucose group (NG, 5.5 mM glucose); AGEs group (AGEs, 0.25mg/mlAGEs); CAL group (CAL, 10, 100nM); CAG group (CAG, 10, 100nM); CAL+CAG group (CAL:CAG, 10µg/ml:5µg/ml, 5µg/ml:10µg/ml); Radix Astragali group (RA, 50 mg/ml aqueous extract). The plates were then incubated for 24 h. Proliferation was examined by MTT reduction assay. Experimental conditions were tested in sextuplicate (six wells of the 96-well plate per experimental condition).

### 3. Results

### 3.1 The effects of CAL and/or CAG on high glucose-induced rat mesangial cell proliferation

As shown in Table 1, compared with the NG group, 25mM glucose (HG) alone increased Rat mesangial cell proliferation after treatment for 24 h (p<0.05). However, addition of CAL and/or CAG, Radix Astragali inhibited high glucose-induced Rat mesangial cell proliferation in a dose-dependent manner at concentrations ranging from 10 to 100 nM. In addition, all the effects of 100nM CAL, 100nM CAG, and CAL+CAG group (CAL:CAG, 5µg/ml:10µg/ml) were better than Radix Astragali group. The effects of CAL+CAG group (CAL:CAG, 5µg/ml:10µg/ml) were better than CAL and CAG groups. These results indicated that CAL and/or CAG could have beneficial effects on early stage DN.

**Table 1 The effects of CAL and/or CAG on high glucose-induced rat mesangial cell proliferation (x̅±s,n=6)**

| Group | Dose | OD value (λ=490nm) |
|---|---|---|
| normal glucose group | -- | 0.35±0.04* |
| high glucose group | -- | 0.43±0.06 |
| Radix Astragali group | 50mg/ml | 0.38±0.02* |
| CAL group | 10nM | 0.34±0.02* |
| | 100nM | 0.33±0.05*^{#} |
| CAG | 10nM | 0.35±0.04* |
| group | 100nM | 0.33±0.03*^{#} |
| CAL:CAG group (2:1) | 10µg/ml:5µg/ml | 0.35±0.05* |
| CAL:CAG group (1:2) | 5µg/ml:10µg/ml | 0.33±0.04*^{#} |

| | | |
|---|---|---|
| *vs* high glucose group * p<0.05; *vs* Radix Astragali group^{#} p<0.05 | | |

### 3.2 The effects of CAL and/or CAG on AGEs-induced rat mesangial cell proliferation

As shown in Table 2, compared with the NG group, 0.25mg/ml AGEs alone increased rat mesangial cell proliferation after treatment for 24 h (p<0.05). However, addition of CAL and/or CAG, and Radix Astragali inhibited AGEs-induced rat mesangial cell proliferation, respectively. In addition, all the effects of CAL and/or CAG were better than Radix Astragali group. The effects of CAL+CAG group were better than CAL group. These results indicated that CAL and/or CAG could have beneficial effects on early stage DN.

**Table 2 The effects of CAL and/or CAG on AGEs-induced rat mesangial cell proliferation (x̅±s, n=6)**

| Group | Dose | OD value(λ=490nm) |
|---|---|---|
| normal glucose group | -- | 0.35±0.04* |
| AGEs group | -- | 0.42±0.05 |
| Radix Astragali group | 50mg/ml | 0.35±0.05* |
| CAL group | 10nM | 0.42±0.04 |
| | 100nM | 0.34±0.04* |
| CAG group | 10nM | 0.34±0.06* |
| | 100nM | 0.33±0.04* |
| CAL:CAG group (2:1) | 10µg/ml:5µg/ml | 0.36±0.05* |
| CAL:CAG group (1:2) | 5µg/ml:10µg/ml | 0.33±0.03* |

| | | |
|---|---|---|
| *vs* AGEs group *p<0.05 | | |

### Experiment 2 The effects of CAL and/or CAG on high glucose induced rat ECM expansion

Expansion and deposition of the mesangial extracellular matrix (ECM) are main pathological features in DN, which is the important target for the treatment of chronic kidney diseases including DN.

### 1. Materials and reagents

TGFβ-1 antibody (Canta Cruz); Di-antibody (Shenzhen Jingmei Bio-engineering Co., Ltd.); SABC Immunohistochemical staining kit (Wuhan Boster Co.); DAB coloration system (Gene Tech Biotechnology Co., Ltd.); Immunohistochemical Box (Fuzhou Maixin Biological Technology Development Co.).

### 2. Method

The treatment was the same as section 2.1 in Experiment 1. The measurement of matrix proliferation was measured by determinating the content of hydroxyproline and the expression of TGFβ-1.

The coverslips were pre-placed in the 24-wells plates to stick the cells. The cells were treated with various concentrations of reagents as indicated in Experiment 1. After the cells covered with coverslip, the supernatant was used for the determination of hydroxyproline and the slides of cells were treated with stained with immunohistochemical and PAS staining. The cells cultured without TGFβ-1 antibody were taken as the negative control.

### 3. Results

### 3.1 The effects of CAL and/or CAG on the content of hydroxyproline in cell culture fluid of rat mesangial cells induced by high glucose

As shown in Table 3, compared with the NG group, 25mM glucose (HG) could increase the content of hydroxyproline in cell culture fluid of rat mesangial cells (p<0.05). However, addition of CAL and/or CAG, and Radix Astragali inhibited the increase of the content of hydroxyproline, respectively. In addition, all the effects of 100nM CAL and 100nM CAG were better than Radix Astragali group. The effects of CAL+CAG group (CAL:CAG, 5µg/ml:10µg/ml) were better than another CAL+CAG group (CAL:CAG, 10µg/ml:5µg/ml).

**Table 3 The effects of CAL and/or CAG on the content of hydroxyproline in cell culture fluid of rat mesangial cells induced by high glucose (x̅±s, n=3)**

| Group | Dose | Content(µg/ml) |
|---|---|---|
| normal glucose group | -- | 4.18±0.37*** |
| high glucose group | -- | 10.06±1.20 |
| Radix Astragali group | 50mg/ml | 7.58±1.95* |
| CAL group | 10nM | 7.15±1.19* |
| | 100nM | 6.65±1.74**^{#} |
| CAG group | 10nM | 6.25±1.20**^{#} |
| | 100nM | 6.00±1.53**^{##} |
| CAL:CAG group (2:1) | 10µg/ml:5µg/ml | 7.05±1.04* |
| CAL:CAG group (1:2) | 5µg/ml:10µg/ml | 6.10±1.13*^{##} |

| | | |
|---|---|---|
| *vs* high glucose group *p<0.05, **p<0.01; *vs* Radix Astragali group ^{#} p<0.05 , ^{##} p<0.01 | | |

### 3.2 The effects of CAL and/or CAG on the ECM accrementition induced by high glucose

As shown in Figs. 1 and 2, addition of CAL and/or CAG, and Radix Astragali inhibited the ECM accrementition and the expression of TGFβ-1induced by high glucose. The effects of CAL or CAG (10, 100nM) were better than Radix Astragali group, and the using CAL and CAG in combination was better than using alone.

From the results of Experiments 1 and 2, the CAL and/or CAG could inhibit rat mesangial cells proliferation, ECM accrementition and TGFβ-1 expression. These results supported the curative effects of CAL and/or CAG on DN.

### Experiment 3 The effects of CAL and/or CAG on diabetic nephropathy model rat induced by streptozotocin

### 1. Materials and reagents

DN model Wistar rats induced by streptozotocin were supplied by animal experiment center of Southern Medical University, BIOBASE-PEARL discrete automatic biochemical analyzer (Shandong BIOBASE Co.).

### 2. Methods

The DN model Wistar rats were randomly divided into 11 groups with 10 rats each group as indicated: normal glucose group; model group; CAL group (6, 2, 0.7mg/kg); CAG group (9, 3, 1mg/kg); CAL+CAG group (CAL:CAG, 2mg/kg:1mg/kg, 1mg/kg:2mg/kg); Radix Astragali group (5g/kg); Aminoguanidine group (100mg/kg).

The drugs or sodium chloride were intragastric adminstrated once each day for 14 weeks. After the administration, the total volume urine was collected by metabolic cage. The contents of urinary production, total protein, microalbumin and creatinine were determinated, and the biochemical indicators in blood was determinated using automatic biochemical analyzer. Then the kideny with formalin fixation was reserved for histopathological examination.

### 3. Results

### 3.1 The effects of CAL and/or CAG on urinary production, total protein, microalbumin and creatinine in STZ DN model rat

As shown in Table 4, intragastric administration of CAL and/or CAG at a moderate or high dose could reduce the contents of urinary production, total protein, microalbumin and creatinine, and the effects of CAL or CAG were better than Radix Astragali group, and the using CAL and CAG in combination was better than or the same as using alone.

**Table 4 The effects of CAL and/or CAG on urinary production, total protein, microalbumin and creatinine in STZ DN model rat (x̅±s, n=10)**

| Group | Dose(mg/kg) | Urinary production (ml) | Urine protein (mg) | Microalbumin (mg) | Urine creatinine (mM) |
|---|---|---|---|---|---|
| Control group | -- | 9.2±1.5** | 18.5±7.7** | 0.29±0.07** | 12.4±2.2** |
| Model group | -- | 65.6±25.3 | 79.4±29.2 | 1.63±0.85 | 1.0±0.3 |
| Aminoguanidine group | 100 | 36.2±21.8* | 43.2±29.9* | 1.00±0.27* | 4.0±2.3** |
| Radix Astragali group | 5000 | 45.3±20.4* | 54.1±24.5* | 1.05±0.52* | 2.1±1.0* |
| | 0.7 | 59.9±21.8 | 69.1±20.4 | 1.57±0.56 | 1.4±0.6 |
| CAL group | 2 | 41.3±16.8* | 54.0±17.4* | 0.93±0.55* | 2.9±1.2** |
| | 6 | 35.9±22.2* | 46.2±21.3*^{#} | 0.92±0.42* | 4.3±2.0**^{##} |
| | 1 | 58.9±20.5 | 67.1±18.4 | 1.52±0.56 | 1.3±0.6 |
| CAG group | 3 | 42.3±12.8* | 55.0±13.4* | 0.90±0.45* | 2.7±1.1** |
| | 9 | 33.9±20.2*^{#} | 44.2±20.6*^{#} | 0.93±0.22* | 4.2±1.6**^{#} |
| CAL:CAG group (2:1) | 3 | 35.3±13.0*^{#} | 50.9±23.7* | 0.89±0.35* | 2.7±0.6** |
| CAL:CAG group (1:2) | 3 | 30.7±24.7*^{#} | 43.5±15.8*^{#} | 0.88±0.26* | 2.8±0.7** |

| | | | | | |
|---|---|---|---|---|---|
| *vs* model group *p<0.05, **p<0.01; *vs* Radix Astragali group: ^{#} p<0.05, ^{##} p<0.01 | | | | | |

### 3.2 The effects of CAL and/or CAG on serum biochemical indicators in STZ DN model rat

As shown in Table 5, intragastric administration of Radix Astragali, CAL and/or CAG could reduce the contents of triglyceride, MDA, Scr, BUN, AGEs and LDL in serum, and significantly increased SOD activity. The effects of CAL or CAG were better than Radix Astragali group, and the using CAL and CAG in combination was better than using alone.

**Table 5 The effects of CAL and/or CAG on serum biochemical indicators in STZ DN model rat (x̅±s, n=10)**

| Group | Dose mg/kg | triglyceride mmol/L | SOD U/ml | MDA nmol/mL | Scr umol/L | BUN mmol/L | AGEs | LDL mmol/L |
|---|---|---|---|---|---|---|---|---|
| Control group | -- | 1.89±0.22** | 525±26** | 4.4±0.7** | 71.7±19.2** | 6.5±0.7** | 264±53** | 0.78±0.30** |
| Model group | -- | 3.36±0.72 | 481±25 | 9.3±2.6 | 96.3±13.4 | 7.9±1.3 | 351±59 | 2.12±0.67 |
| Aminogua nidine group | 100 | 3.35±0.87 | 516±20** | 5.9±1.7** | 77.7±20.3* | 6.6±0.8* | 283±78* | 2.01±0.58 |
| | 0.7 | 3.23±0.76 | 498±33 | 7.8±2.3 | 91.0±31.1 | 7.0±1.0 | 290±59 | 1.91±0.55 |
| CAL group | 2 | 2.76±0.80* | 513±41 | 6.8±1.9* | 76.6±24.5* | 6.6±1.2* | 289±56* | 1.51±0.53* |
| | 6 | 2.57±0.80* | 520±29** | 6.8±1.3* | 75.2±17.1** | 6.5±1.5* | 281±78* | 1.43±0.36* |
| | 1 | 3.20±0.71 | 490±23 | 7.9±1.6 | 89.0±21.1 | 6.9±0.9 | 288±44 | 1.94±0.44 |
| CAG group | 3 | 2.66±0.72* | 502±31* | 6.5±1.4* | 73.5±22.5* | 6.7±0.8* | 281±46* | 1.60±0.43* |
| | 9 | 2.43±0.70* | 512±19** | 6.5±1.2* | 74.2±16.0** | 5.9±1.6* | 275±68* | 1.23±0.26* |
| CAL:CAG group (2:1) | 3 | 2.81±0.77* | 503±24* | 6.7±1.5* | 76.8±23.4* | 6.9±1.0 | 278±51* | 1.47±0.45* |
| CAL:CAG group (1:2) | 3 | 2.64±0.75* | 500±27* | 6.6±1.1* | 73.0±23.9** | 6.2±0.9* | 280±50* | 1.43±0.32* |

| | | | | | | | | |
|---|---|---|---|---|---|---|---|---|
| *vs* model group *p<0.05, **p<0.01; SOD: superoxide dismutase; MDA: malonaldehyde; Scr: serum creatinine; BUN: Blood urea nitrogen; AGEs: advanced glycosylation end products; LDL: terminal glycosylation lipids. | | | | | | | | |

### 3.3 The effects of CAL and/or CAG on pathohistological indicators in STZ DN model rat

As shown in Table 6, intragastric administration of Radix Astragali, CAL and/or CAG could significantly inhibit the ECM accrementition and TGFβ-1 expression, and reduce the pathological damage score. The effects of CAL or CAG were better than Radix Astragali group, and the using CAL and CAG in combination was slightly better than using alone.

**Table 6 The effects of CAL and/or CAG on pathohistological indicators in STZ DN model rat (x̅_{±s,} n=10)**

| Group | Dose(mg/kg) | TGFβ-1(%) | Patho-score | ECM score(PAS%) |
|---|---|---|---|---|
| Control group | -- | 3.4±1.5** | 0.3±0.5** | 22.1±4.4** |
| Model group | -- | 23.7±5.6 | 3.4±1.3 | 36.2±3.6 |
| Aminoguanidine group | 100 | 11.7±5.7** | 2.1±1.0* | 26.5±4.3** |
| Radix Astragali group | 5000 | 16.3±5.4* | 1.9±2.0* | 32.8±4.3 |
| | 0.7 | 19.3±6.3* | 2.7±1.0 | 33.1±5.1 |
| CAL group | 2 | 17.6±5.8* | 1.8±1.3* | 29.8±5.6** |
| | 6 | 8.8±3.2** | 1.6±1.1** | 27.0±5.5** |
| | 1 | 20.0±4.3* | 2.8±0.9 | 32.5±4.1 |
| CAG group | 3 | 16.2±4.7* | 1.9±1.1* | 28.9±5.4** |
| | 9 | 9.1±2.2**^{#} | 1.7±0.9** | 26.9±4.5** |
| CAL:CAG group (2:1) | 3 | 17.4±4.0* | 2.0±1.6* | 29.1±5.5** |
| CAL:CAG group (1:2) | 3 | 12.9±3.6**^{#} | 1.8±0.9* | 27.3±4.7** |

| | | | | |
|---|---|---|---|---|
| *vs* model group *p<0.05, **p<0.01; *vs* Radix Astragali group^{#} p<0.05 | | | | |

In all, all the above experiment results showed that CAL and/or CAG could show prevention and treatment of the occurrence and development of DN, and protective effects on STZ DN model rat. In addition, they also could reduce the level of renal oxidative stress and fibrosis caused by AGEs, and the effect was superior to aqueous extract of Radix Astragali.

## Claims

1. A pharmaceutical composition for use in preventing and treating diabetic nephropathy, comprising as active ingredient both calycosin and calycosin-7-O-β-D-glucoside, and the conventional drug carrier, wherein the weight ratio of calycosin to calycosin-7-O-β-D-glucoside is in a range from 1:5 to 5:1, the active ingredient is in a percentage of 0.1- 99.5% by weight.

2. The pharmaceutical composition for use according to claim 1, wherein the conventional drug carrier comprises at least one of diluent, excipient, filler, adhesive, wetting agent, disintegrant, absorption enhancer, surfactant, adsorption carrier, lubricant, flavor agent and sweetener, wherein the excipient is water ; the filter is starch, sucrose or lactose; the adhesive at least one selected from a group consisting of cellulose derivative, alginate, gelatin and polyvinylpyrrolidone; the wetting agent is glycerine; the disintegrant is agar, calcium carbonate or sodium bicarbonate; the absorption enhancer is quaternary ammonium compound ; the surfactant is palmityl alcohol; the adsorption carrier is kaolin clay or soap clay; the lubricant is talc, calcium stearate, magnesium stearate or polyethylene glycol.

3. The pharmaceutical composition for use according to claim 2, wherein the dosage form of pharmaceutical composition is tablet, granule, pill, powder, capsule or liquid formulation.

4. A method for preparing the pharmaceutical composition of claim 1, comprising: mixing both calycosin and calycosin-7-O-β-D-glucoside with the conventional drug carrier to obtain the pharmaceutical composition.

5. The method of claim 4, wherein the preparation of the calycosin and calycosin-7-O-β-D-glucoside comprises the following steps:
A. Radix Astragali in pulverization is extracted with 80% ethanol under reflux and filter, and the alcohol extracts are combined and then concentrated in vacuo, the concentrates are then refrigerated overnight;
B. the supernatant obtained by step A is run on a macroporous resin column and eluted with water, and then 50% ethanol until no calycosin-7-O-β-D-glucoside is detected using TLC;
C. the 50% ethanol fraction solvent is concentrated, then extracted using equivalent ethyl acetate, and the ethyl acetate extracts are combined and then concentrated in vacuo;
D. the extract is chromatographed over silica gel, and eluted gradiently with a solvent system of chloroform-methanol, and the same fractions are combined by TLC analysis in order to crystallize, then the crystal is obtained with sucking filtration, calycosin and calycosin-7-O-β-D-glucoside are obtained by recrystallization in methanol.

6. The use of calycosin and calycosin-7-0-ß-O-glucoside in the manufacture of a medicament for the prevention and treatment of diabetic nephropathy, wherein the weight ratio of calycosin to calycosin-7-O-β-D-glucoside is in a range from 1:5 to 5:1.

## Patentansprüche

1. Pharmazeutische Zusammensetzung zur Verwendung beim Verhindern und Behandeln diabetischer Nephropathie, als Wirkstoff sowohl Calycosin als auch Calycosin-7-O-β-D-glucosid und den herkömmlichen Arzneimittelträger umfassend, wobei das Gewichtsverhältnis von Calycosin zu Calycosin-7-0-β-D-glucosid in einem Bereich von 1:5 bis 5:1 liegt, wobei der Wirkstoff zu einem Prozentsatz von 0,1 bis 99,5 Gew.-% vorliegt.

2. Pharmazeutische Zusammensetzung zur Verwendung nach Anspruch 1, wobei der herkömmliche Arzneimittelträger mindestens eines des Folgenden umfasst: ein Streckmittel, einen Hilfsstoff, einen Füllstoff, einen Klebstoff, ein Benetzungsmittel, ein Sprengmittel, einen Absorptionsverstärker, ein Tensid, einen Adsorptionsträger, ein Gleitmittel, Aromastoff und Süßungsmittel, wobei der Hilfsstoff Wasser ist, der Füllstoff Stärke, Sucrose oder Lactose ist, der Klebstoff mindestens einer ist, der aus einer Gruppe ausgewählt ist, die besteht aus: Cellulosederivat, Alginat, Gelatine und Polyvinylpyrrolidon, das Benetzungsmittel Glycerin ist, das Sprengmittel Agar, Calciumcarbonat oder Natriumbicarbonat ist, der Absorptionsverstärker eine quaternäre Ammoniumverbindung ist, das Tensid Palmitylalkohol ist, der Adsorptionsträger kaolnitischer Ton oder Bentonit ist, das Gleitmittel Talk, Calciumstearat, Magnesiumstearat oder Polyethylenglycol ist.

3. Pharmazeutische Zusammensetzung zur Verwendung nach Anspruch 2, wobei die Darreichungsform der pharmazeutischen Zusammensetzung eine Tablette, Granulat, eine Pille, Pulver, eine Kapsel oder eine flüssige Formulierung ist.

4. Verfahren zur Herstellung der pharmazeutischen Zusammensetzung nach Anspruch 1, Folgendes umfassend: Mischen von sowohl Calycosin als auch Calycosin-7-0-β-D-glucosid mit dem herkömmlichen Arzneimittelträger, um die pharmazeutische Zusammensetzung zu erhalten.

5. Verfahren nach Anspruch 4, wobei die Herstellung des Calycosins und Calycosin-7-0-β-D-glucosids die folgenden Schritte umfasst:
A Radix Astragali in Pulverform wird mit 80%igem Ethanol unter Rückfluss und Filterung extrahiert und die Alkoholextrakte werden kombiniert und dann unter Unterdruck konzentriert, die Konzentrate werden dann über Nacht tiefgekühlt,
B der in Schritt A erzielte Überstand wird in eine makroporöse Harzsäule geleitet und mit Wasser und danach mit 50%igem Ethanol eluiert, bis bei einer TLC kein Calycosin-7-0-β-D-glucosid erkannt wird,
c das Lösemittel der 50%igen Ethanolfraktion wird konzentriert und dann unter Verwendung eines äquivalenten Ethylacetats extrahiert, die Ethylacetatextrakte werden kombiniert und dann unter Unterdruck konzentriert,
D der Extrakt wird über Siliziumoxidgel chromatographiert und schrittweise mit einem Lösemittelsystem aus Chloroformmethanol eluiert, die gleichen Fraktionen werden durch TLC-Analyse kombiniert, um zu kristallisieren, dann wird das Kristall mittels Saugfilterung erzielt, Calycosin und Calycosin-7-0-β-D-glucosid werden durch Rekristallisierung in Methanol erzielt.

6. Verwendung von Calycosin und Calycosin-7-0-β-D-glucosid bei der Herstellung eines Medikaments zur Verhinderung und Behandlung von diabetischer Nephropathie, wobei das Gewichtsverhältnis von Calycosin zu Calycosin-7-0-β-D-glucosid in einem Bereich von 1:5 bis 5:1 liegt.

## Revendications

1. Composition pharmaceutique destinée à être utilisée dans la prévention et le traitement d'une néphropathie diabétique, comprenant comme substances actives aussi bien la calycosine que le calycosine-7-O-5-D-glucoside, ainsi que le vecteur de médicament conventionnel, dans lequel le rapport massique de la calycosine par le calycosine-7-O-β-D-glucoside est compris dans une plage de 1:5 à 5:1 et la substance active est un pourcentage compris entre 0,1 % et 99,5 % en masse.

2. Composition pharmaceutique 2 destinée à une utilisation selon la revendication 1, dans laquelle le vecteur de médicament conventionnel comprend au moins un composé parmi un diluant, un excipient, une substance de remplissage, un adhésif, un agent de mouillage, un désintégrant, un promoteur d'absorption, un surfactant, un support d'adsorption, un lubrifiant, un agent aromatique et un édulcorant, dans lequel l'excipient est de l'eau ; la substance de remplissage est de l'amidon, du saccharose ou du lactose ; l'adhésif est au moins un adhésif sélectionné parmi un groupe constitué par les dérivés de la cellulose, les alginates, la gélatine et la polyvinylpyrrolidone ; l'agent de mouillage est la glycérine ; le désintégrant est de l'agar-agar, du carbonate de calcium ou du bicarbonate de sodium ; le promoteur d'absorption est un composé ammonium quaternaire ; le surfactant est l'alcool palmitique ; le support d'adsorption est de l'argile kaolinite ou de l'argile à savon ; le lubrifiant est du talc, du stéarate de calcium, du stéarate de magnésium ou du polyéthylène glycol.

3. Composition pharmaceutique 2 destinée à une utilisation selon la revendication 2, dans laquelle la forme galénique de la composition pharmaceutique est un comprimé, un granule, une pilule, une poudre, une capsule ou un liquide.

4. Procédé de préparation de la composition pharmaceutique selon la revendication 1, comprenant : le mélange de calycosine ainsi que de calycosine-7-O-β-D-glucoside avec le vecteur de médicament conventionnel pour obtenir la composition pharmaceutique.

5. Procédé selon la revendication 4, dans lequel la préparation de la calycosine et de la calycosine-7-O-β-D-glucoside comprend les étapes suivantes :
A. de la racine d'astragale, soit Radix Astragali, pulvérisée est extraite avec de l'éthanol à 80 % par reflux et filtration, et les extraits d'alcool sont combinés puis concentrés sous vide, après quoi les concentrés sont réfrigérés durant une nuit ;
B. le surnageant obtenu à l'étape A est passé sur une colonne de résine macroporeuse et élué avec de l'eau, puis avec de l'éthanol à 50 % jusqu'à ce que le calycosine-7-O-β-D-glucoside ne soit plus détecté par chromatographie sur couche mince ;
C. la fraction sous solvant éthanol à 50 % est concentrée puis extraite à l'aide d'acétate d'éthyle équivalent, et les extraits d'acétate d'éthyle sont combinés puis concentrés sous vide ;
D. l'extrait est traité par chromatographie sur gel de silice et élué par gradient avec un système de solvants chloroforme-méthanol, et les mêmes fractions sont combinées par analyse à chromatographie sur couche mince de manière à cristalliser, puis le cristal est obtenu par filtration à aspiration, et la calycosine et le calycosine-7-O-β-D-glucoside sont obtenus par recristallisation dans du méthanol.

6. Utilisation de calycosine et de calycosine-7-O-β-D-glucoside dans la fabrication d'un médicament pour la prévention et le traitement d'une néphropathie diabétique, dans laquelle le rapport massique de la calycosine par le calycosine-7-O-β-D-glucoside est compris dans une plage de 1:5 à 5:1.
